# EUROPEAN PATENT SPECIFICATION

(11) **EP 0 877 022 B1**
(45) Date of publication and mention of the grant of the patent: **16.04.2003**
(21) Application number: 98108370.2
(22) Date of filing: 07.05.1998
(51) Int. Cl.: C07D 239/54, C07D 401/12, C07D 239/46, C07D 493/04, C07D 489/04, A61K 31/505

(54) **Sulfonylpyrimidine derivatives with anticancer activity**
Sulfonylpyrimidine Derivate mit Antitumor Wirkung
Dérivés de sulfonylpyrimidine avec une action antitumorale

(30) Priority: 09.05.1997 HR 970239
(43) Date of publication of application: 11.11.1998
(73) Proprietor: Rudjer Boskovic Institute, 10001 Zagreb (HR)
(72) Inventor: Zinic, Biserka, Dr., 11001 Zagreb (HR); Zinic, Mladen, Dr., 11001 Zagreb (HR); Krizmanic, Irena, 44000 Sisak (HR)
(74) Representative: Viering, Jentschura & Partner

(56) References cited:
- DE-A- 2 455 423
- GB-A- 1 570 555
- CHEMICAL ABSTRACTS, vol. 107, no. 15, 1987 Columbus, Ohio, US; abstract no. 134623s, page 756; XP002077372 & JP 62 093281 A (KYOWA HAKKO KOGYO CO)
- CHEMICAL ABSTRACTS, vol. 105, no. 1, 1986 Columbus, Ohio, US; abstract no. 218430y, M.KALDRIKYAN ET AL.: "SYNTHESIS A.BIOLOGICAL ACTIVITY OF N'-4-ALKOXY-BENZENESULFONYL-5-HALOURACILS. " page 29; XP002077373 & KHIM. FARM. ZH., vol. 20, no. 8, 1986, pages 928-932, USSR
- CHEMICAL ABSTRACTS, vol. 94, no. 17, 1981 Columbus, Ohio, US; abstract no. 139826h, page 768; XP002077374 & JP 55 087 777 A (ONO) 02 July 1980
- CHEMICAL ABSTRACTS, vol. 83, no. 28, 1975 Columbus, Ohio, US; abstract no. 9973r, TADA,MASSO: "ANTINEOPLASTIC AGENTS." page 835; XP002077375 & CHEM. LETT., vol. 2, 1975, pages 129-130, JAPAN
- J.YAMASHITA ET AL.: "STUDIES ON ANTITUMOR AGENTS.V." CHEMICAL AND PHARMACEUTICAL BULLETIN., vol. 30, no. 12, 1982, pages 4258-4267, XP002077368 TOKYO JP
- CHEMICAL ABSTRACTS, vol. 86, no. 13, 1977 Columbus, Ohio, US; abstract no. 89867j, page 550; XP002077376 & JP 51 105 080 A (MITSUI SEIYAKU) 17 September 1976
- T.KURODA ET AL.: "G-RADIOLYSIS OF 5-F.URACIL AND 5-F.URIDINE DERIVATIVES" JOURNAL OF HETEROCYCLIC CHEMISTRY., vol. 31, no. 2, March 1994, pages 335-339, XP002077369 PROVO US
- H. BALLWEG: "EINE NEUE METHODE ZUR DARSTELLUNG VON CYTOSINE" TETRAHEDRON LETTERS., vol. 18, 1968, pages 2171-2173, XP002077370 OXFORD GB
- B.KASNAR ET AL.: "SYNTHESIS OF THE SULFONYLPYRIMIDINE DERIVATIVES" NUCLEOSIDES & NUCLEOTIDES., vol. 16, July 1997, pages 1067-1071, XP002077371 INC US

## Description

### 1) FIELD OF THE INVENTION

This invention relates to the N-1 sulfonyl derivatives of pyrimidine nucleo bases having anticancer properties, to processes of their preparation and to anticancer activity of such compounds.

### 2) TECHNICAL PROBLEM

The use of sulfonylurea derivatives is known in medicinal chemistry and agrochemistry. These compounds exhibit very interesting biological activities: antidiabetic, antibacterial and anticancer effect, as well as very strong herbicidal effect. Recently, sulfonylurea herbicides represent remarkable improvement due to their high activity and almost none human and animal toxicity.

On the other hand, there is quite a number of biologically active nucleoside derivatives with antibiotic, cancerostatic and virostatic effects. Modified nucleosides are the only compounds having *in vitro* and *in vivo* antiviral and anti-HIV activity.

In the case of unknown N-1 sulfonyl derivatives of pyrimidine nucleo bases representing a new type of sulfonylcycloureas, there is an interesting combination of pharmacophores, which could result in antiviral and anticancer activity, and lead to development of new chemotherapeutics.

### 3) BACKGROUND OF THE INVENTION

Sulfonylureas are compounds with strong antibacterial (F. Kurzer, *Chem. Rev.* **50** (1952) 1.) and hypoglycemic activity (*Chem. Abstr.* **102** (1985) 214964r; G. Levitt, *US 4.435.206*; *Chem. Abstr.* **100** (1984) 34458d; *Chem. Abstr.* **100** (1984) 34459e; L. Aguilar-Bryan, D. A. Nelson, Q. A. Vu, M. B. Humphrey, and A. E. Boyd,. *J. Biol. Chem.* **265** (1990) 8218.; *Chem. Abstr.* **108** (1988) 161258d; *Chem. Abstr.* **106** (1987) 149243w; A. G. Hatzidimitriou, D. P. Kessissoglou, and G. E. Manoussakis, *J. Inorg. Biochem.* 49 (1993) 157.). They are strong diuretics (*Chem. Abstr.* ***103*** (1985) 215193w, *EP-A1-151.451*), antitumor agents (*Chem. Abstr.* **108** (1988) 94225a, *EP-A1-222.475*; F. Mohamadi, M. M. Spels, and G. B. Grindey, *J. Med. Chem.* **35** (1992) 3012.; J. J. Howbert, C. S. Grossman, T. A. Crowell, B. J. Rieder, R. W. Harper, K. E. Kramer, E. V. Tao, J. Aikins, G. A. Poore, *et.* *al, J*. *Med*. *Chem.* **33** (1990) 2393; *Chem. Abstr.* **120** (1994) 245006a, *EP-A2-2.583.960*) and anticholesteremics (D. R. Sli 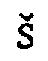 ković, B. R. Krause, J. A. Picard, M. Anderson, R. F. Bousley, K. L. Hamelehle, R. Homan, T. N. Julian, Z. A. Rashidbaigi, and R. L. Stanfield, *J. Med. Chem.* (1994) 560; *Chem Abstr.* **119** (1993) 138915j, *WO-A1-9.308.161*; *P.* R. Carter, D. H. Hey, and D. S. Morris, *J. Chem Soc.* (1948) 143.). Sulfonylureas having heteroaromatic (Het) groups bound to NH of sulfonylurea bridge (R-SO₂-NH-CO-NH-**Het**) are extremely potent herbicides.

New sulfonylcycloureas, *e.g.* N-1 sulfonyl derivatives of uracil and cytosine, are not known, neither are their biological activities. There was only Martirosyan (Z. A. Martirosyan, V. I. Gundar, and S. L. Zav'yalov, *Izv. Akad. Nauk SSSR, Ser. Khim.* **8** (1970) 1841) who isolated 1-*p*-toluenesulfonyluracil as unwanted product in the transformation of C-4 keto group of uracil. From the view of biological activity, N-1 sulfonyl derivatives of pyrimidine nucleo bases are very interesting compounds since they have a combination of biological active components: a sulfonylurea fragment and a nucleo base. We have prepared the new, unknown sulfonylcycloureas by binding the sulfonyl fragment on N-1 atom of pyrimidine bases (uracil, cytosine or 5-azauracil). These compounds could potentially have biological properties in the sense of their similar structural features to active sulfonylureas that quite possibly have sixmembered hydrogen bonded pseudocycle. On the other hand, one can consider these compounds modified nucleosides with potential anticancer activity *(Chemistry, Biology, and Clinical Uses of Nucleoside Analogs, A.* Bloch (Ed.), New York, 1975, p. 185.), as well as antiviral activity (E. de Clercq, in: *Design of Anti-AIDS Drugs,* E. de Clercq (Ed.), Amsterdam, Elsevier Science Publishers, B. V., 1990, p.1; H. Mitsuya, T. Shirasaka, and S. Broder, in: *Design of Anti-AIDS Drugs,* E. de Clercq (Ed.), Amsterdam, Elsevier Science Publishers, B. V., 1990, p. 25.).

This invention shows that N-1 sulfonyl derivatives of pyrirnidine nucleo bases have remarkable anticancer activity *in vitro* on different tumor cell lines.

GB 1,570,555 discloses anticancer compositions comprising a 5-fluorouracil core providing strong anticancer effects but suppressed toxicity and side effects. Among useful 5-fluorouracils are compounds like 1-benzenesulfonyl-5-fluorouracil and 1-(p-toluenesulfonyl)-5-fluorouracil. Furthermore, it is noted that said anticancer compositions when administered, provide an exceedingly increased 5-fluorouracil concentration in cancer tissues, whereas other tissues, such as the blood serum, will exhibit little or no increase in the concentration of 5-Fluorouracil. According to GB 1,570,555, a variety of different N-substituents are suitable.

The prior art document, DE 24 55 423 is drawn to 5-fluorouracil-derivatives, processes for the manufacture thereof and pharmaceutical compositions comprising said 5-fluorouracil derivatives. DE 24 55 423 provides 5-fluorouracil derivatives with novel N-substituents compared to those disclosed in GB 1,570,555. These fluorouracil derivatives provide a decreased toxicity but nevertheless increased anticancer activity.

JP-A-62093281 describes the preparation of fluoropyrimidine and fluoropyrimidine nucleoside derivatives as antitumor agents. Among other types of compounds, 5-fluorouracil derivatives with varying N-substituents are disclosed. The administration of 1000 µg inside the tumor in mice transplanted with leukemia P388 cells, followed by the irradiation of said mice under ¹³⁷Cs at 1000 R results in a reduced tumor size of 47% over the control after 7 days.

Kaldrikyan et al., (Chemical Abstracts, Vol. 105, nº1, 1986, abstract nº218430Y) reports about the synthesis and biological activity of N'-4-alkoxy-benzenesulfonyl-5-halouracils. Especially the bromo derivatives tended to exhibit antibacterial activity in mice and rats, whereas the fluoroderivatives exhibited antitumor, radioprotective and antimutagenic properties. Elongation of the alkoxy radical results in increased antitumor activity, whereas the antimutagenic activity was negatively correlated.

JP-A-80087777 describes uracil derivatives as auxiliaries for antitumor 5-fluorouracil compositions. The addition of such an uracil derivative enhanced antitumor activity of N'-ethoxymethyl-5-fluorouracil, N'-(2-furanidyl)-5-fluorouracil and similar compounds. The combined use of these compounds has shown a good activity against sarcoma 180 in mice.

Tada et al, discloses antineoplastic agents (Chem. Abs., vol. 83, nº 28, 1975, abstract 99735). It is reported about the synthesis of 1-substituted-5-fluorouracil derivatives. According to Tada et al., 1975, benzoyl and arene-sulfonyl-fluorouracils are more active and less toxic than 1-(2-tetrahydrofuryl)uracil in Leukemia L-1210 system.

Kuroda et al., (Journal of Heterocyclic Chemistry, Vol. 31, nº2 March 1994, 335-339) reports about γ-Radiolysis of 5-fluorouracil and 5-fluorouridine derivatives having sulfur-containing substituents. Due to their specific radiolysis properties, 5-fluorouracil derivatives bearing a variety of sulfonyl group containing substituents and 5-fluorouridine derivatives bearing a variety of thioureido group containing substituents and might be potential radio-induced drugs for cancer therapy.

JP-A-76105080 describes 5-fluorouracil derivatives, although no hint for any potential anticancer activity is given.

Yamashita et al., (Chem. Pharm. Bull., vol. 30, nº12, 1982, 4258-4267) provides studies on the synthesis and antitumor activities of 5-fluorouracil derivatives. After oral administration of these derivatives to rats, the blood levels of 5-fluorouracil were determined. Among O-substituted derivatives, a 4-O-substituted derivative was most easily activated to 5-fluorouracil, and 2-O-substituted derivatives were second most easily activated. Among N-substituted derivatives, acyl and sulfonyl derivatives showed the highest 5-fluorouracil releasing abilities and 1-alkoxymethyl substituted derivatives showed low ability. N-alkyl substituted derivatives were not activated to 5-fluorouracil.

Ballweg et al., (Tetrahedron Letters, Vol. 18, 1968, 2171-2173) describes a novel method for synthezising cytosin and cytosin derivatives. According to Ballweg et al., 1967, cytosin and 3-methyl cytosin can be provided in a simpler manner than previous synthetic procedures.

Kasnar et al., (Nucleosides & Nucleotides, Vol. 16, 1997, 1067-1071) reports about the synthesis of sulfonylpyrimidine derivatives as a new type of sulfonylcycloureas. The synthesis of several novel N-1 and N-1,NH-4-disulfonylpyrimidine derivatives are described. These studies were directed to the synthesis of novel sulfonylurea derivatives as potential herbicides. For the synthesis of sulfonylpyrimidine derivatives, two methods were used: a) condensation of silylated pyrimidine bases with different sulfonyl chlorides in acetonitrile and b) reaction of pyrimidine bases with sulfonyl chlorides in pyridine. The preliminary testing of some of those compounds revealed herbicide activity, however, there is no indication that these compounds show any antitumor activity.

### 4) SUMMARY OF THE INVENTION

This invention relates to N-1 sulfonyl derivatives of pyrimidine nucleo bases of general formulae II, to the processes of their preparation and their biological activity. 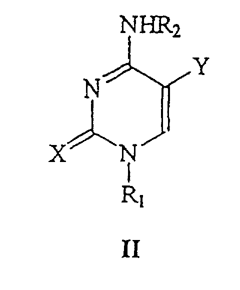 In general formula II:
X = O,S;
Y = H, F, Cl, Br, I, C₁-C₅ alkyl, NO₂, NH₂, substituted alkynyl (C≡CR₄; R₄ = H, Si(CH₃)₃, C₁-C₃ alkyl, halogen, CO-alkyl); substituted alkenyl (CH=CHR₅; R₅ = H, F, Cl, Br, I, C₁-C₃ alkyl, CN, COCH₃, CO₂CH₃);
R₁ = SO₂R₆;
R₂ = H or SO₂R₆ in which R₆ is the same or different when R₁ = SO₂R₆ and R₂ = SO₂R₆
R₆ = C₁-C₃ (branched) alkyl, C₁-C₃ haloalkyl, C₁-C₃ alkyl substituted with C₁-C₃ (halo)alkoxy group, C₂-acyl,

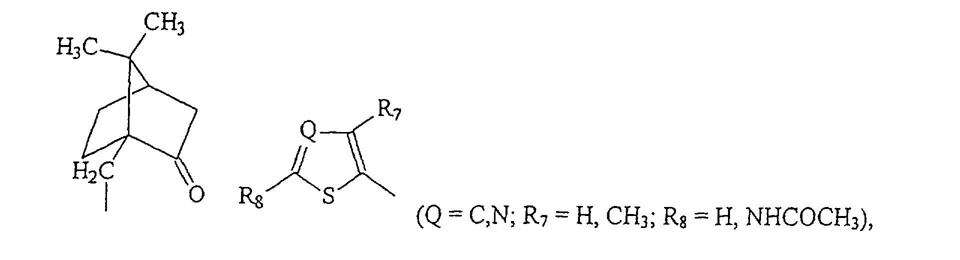

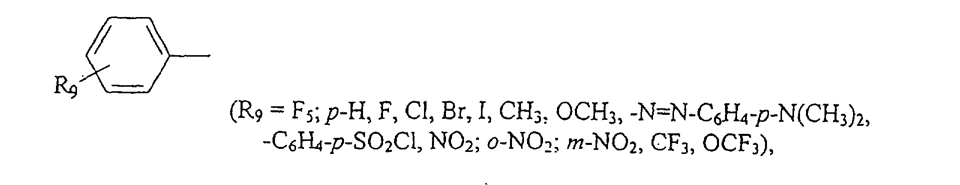

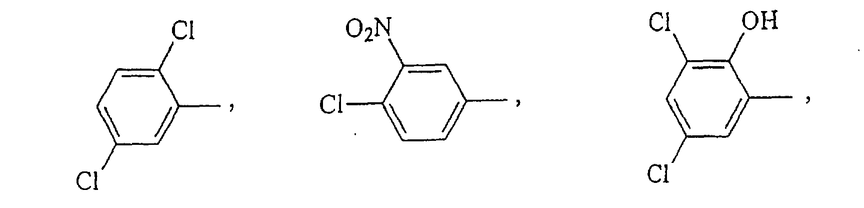

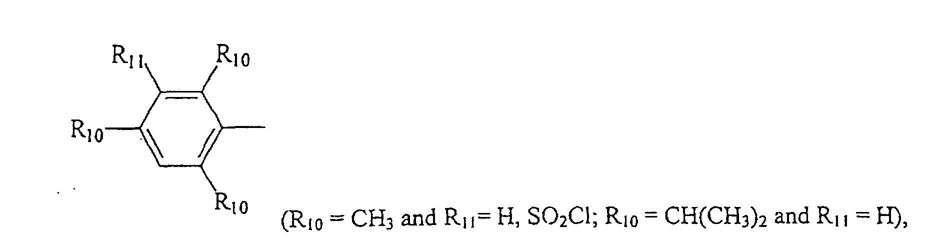

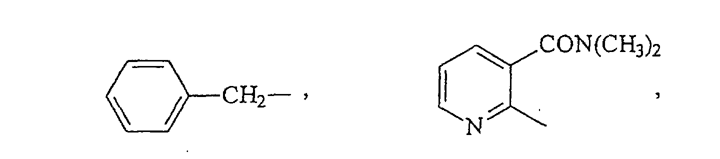

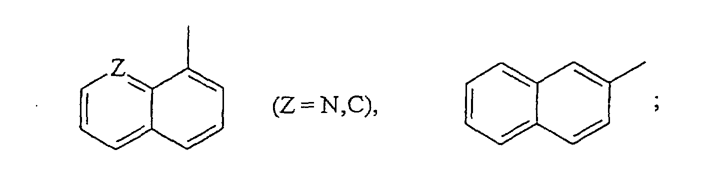

For the synthesis of sulfonylpyrimidine derivatives we have used two methods:
**a)** condensation of silylated pyrimidine bases with different sulfonyl chlorides in acetonitrile;
**b)** reaction of pyrimidine bases with sulfonyl chlorides in pyridine.

The compounds of general formula **II** were prepared according to Scheme (1a) (J. R. Hanson, A. Hough, and A. T. White, *J. Chem. Soc.; Chem. Commun.* (1968) 466; J. J. de Koning, H. J. Kooreman, H. S. Tan, and J. Verweij, *J. Org. Chem.* **40** (1975) 1346.). Derivatives of cytosine are sylilated with BSA in dry acetonitrile, under reflux for 30 minutes, according to method **a).** Such activated compounds react with appropriate sulfonyl chlorides at 20 °C to reflux, for 1 hour, giving N-1 sulfonyl derivatives of general formula **II** in 40-80% yield, Scheme (1a).

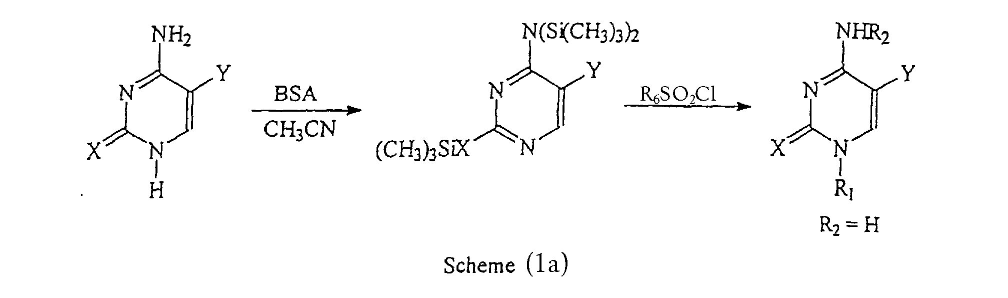

N-1,NH-4. Disulfonyl derivatives of cytosine **II** can be obtained according to method b) (Scheme (1b): R₁ = R₂ = SO₂R₆ (R₆ = as in II). In the reaction of cytosine with 2 eq. of sulfonyl chlorides ClSO₂R₆ in pyridine, compounds of general formula **II** with R₁ = R₂ were obtained. In the reaction of N-1 sulfonyl derivatives of cytosine with different sulfonyl chlorides ClSO₂R₆ compounds of general formula **II** with R₁ ≠ R₂ were obtained.

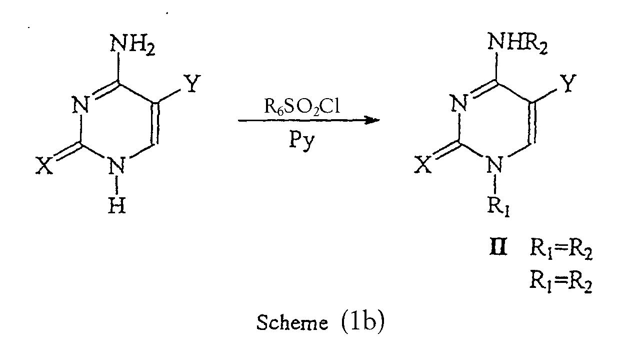

C-5 Halogen derivatives of cytosine can be prepared according to known procedures (J. Duval and J. P. Ebel, *Bull. Soc. Chim. Biol.* **46** (1964) 1059; P. K Chang, in: *Nucleic Acid Chemistry*, Part 2, L. B. Townsend and R. S. Tipson (Eds.), New York, John Wiley & Sons, 1978, p. 779.).

Compounds 1 and 2, as shown in the Scheme (2) were tested *in vitro* on human tumor pancreatic carcinoma cells (MiaPaCa2), human cervix carcinoma cells (HeLa), laryngeal carcinoma cells (Hep 2) and normal human fibroblasts (Hef 2).

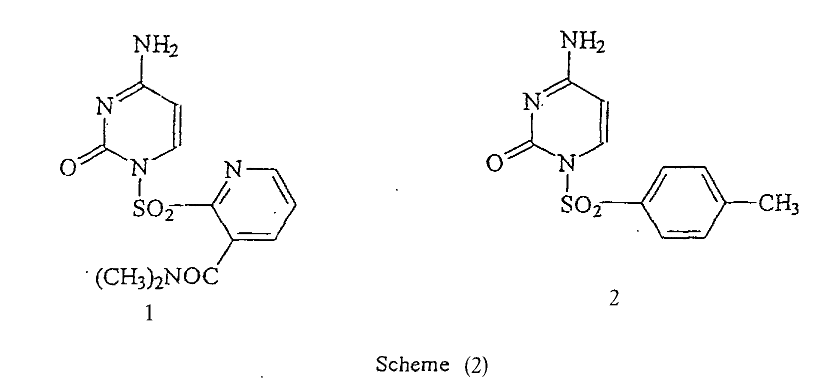

All tested compounds show remarkable growth inhibitory effects on the tumor cell lines MiaPaCa 2 and HeLa (60-90%, in the concentration 10⁻⁵ M) and much less inhibition on normal cell lines (20-35%). The most remarkable inhibition effect on Hep 2 cell lines was found for compound 1 (80%) in the concentration 10⁻⁵ M.

### EXAMPLE 1. 1-p-Toluenesulfonylcytosine

The mixture of cytosine (222 mg, 2.00 mmol) and BSA (1.48 ml, 6.00 mmol) was heated under reflux in dry acetonitrile (7 ml) for 30 minutes. The solution was cooled to 0 °C and TsCl (457 mg, 2.40 mmol) was added. After heating under reflux for 45 minutes, the solvent was evaporated. Ethanol was added in the residue and the resulting solid was filtered and recrystallized from hot ethanol yielding white crystals of the product (421 mg, 80%), mp 216 °C; UV (MeOH): λₘₐₓ/nm 209.1 and 248.0 (log ε 3.52 and 3.57); IR (KBr) *ν*_{*max*}/cm⁻¹: 3360 (s), 3100 (s), 1660 (s, br), 1550 (s), 1490 (s), 1380 (s), 1355 (s), 1285 (s), 1185 (s), 1175 (s); ¹H-NMR (d₆-DMSO) δ/ppm: 8.12 (d, 1H, H-6, J=7.94 Hz), 7.94 (s, 2H, NH₂), 7.86 (d, 2H, Ph, J=8.24 Hz), 7.45 (d, 2H, Ph, J=8.17 Hz), 5.96 (d, 1H, H-5, J=7.94 Hz), 2.41 (s, 3H, CH₃); ¹³C-NMR (d₆-DMSO) δ/ppm: 166.27 (s, C-4), 151.22 (s, C-2), 145.61 (d, C-6), 139.73 (s, Ph), 134.47 (s, Ph), 129.80 (d, Ph), 129.02 (d, Ph), 97.50 (d, C-5), 21.20 (q, CH₃); *Anal.* Calcd. for C₁₁H₁₁N₃O₃S (*M*ᵣ*=*265.30): C (49.80), H (4.18), N (15.84), found: C (50.09), H (4.02), N (15.89).

### EXAMPLE 2. 1-(2-Sulfonyl-3-N,N-dimethylaminocarbonylpyridine)cytosine

### The condensation of cytosine with sulfonyl-chloride

The mixture of cytosine (191 mg, 1.71 mmol) and BSA (1.28 ml, 5.15 mmol) in dry acetonitrile (6 ml) was heated under reflux for 30 minutes. The colorless solution was cooled to 0 °C and put on the sulfonyl-chloride [prepared as those for uracil derivative, starting from 1.06 g (3.89 mmol) of 2-phenylmethylthio-3-*N,N*-dimethylaminocarbonylpyridine]. After stirring at room temperature for 2 hours, the solvent was evaporated under reduced pressure without external heating. Methanol was added to the remaining oil and white solid of the product was filtered and recrystallized from methanol (290 mg, 53%), mp 218 °C; UV (MeOH): λₘₐₓ/nm 246.1 (log ε 4.07); IR (KBr) νₘₐₓ/cm⁻¹: 3400 (m), 3140 (v, br), 2920 (vw), 1700 (s), 1510 (m), 1490 (s), 1390 (s), 1360 (s), 1280 (m), 1170 (s); ¹H-NMR (d₆-DMSO) δ/ppm: 8.68 (d, 1H, H-6', *J*=4.6 Hz), 8.06 (m, 4H, H-4', H-6, NH₂), 7.78 (dd, 1H, H-5', *J*_{5',6'}=4.6 Hz, *J*_{5',4'}=7.6 Hz), 6.04 (d, 1H, H-5, *J*=7.9 Hz), 2.99 (s, 3H, NCH₃), 2.89 (s, 3H, NCH₃); ¹³C-NMR (d₆-DMSO) δ/ppm: 166.43 (s, C-2'), 165.47 (s, C-4), 151.96 (s, C=O), 150.86 (s, C-2), 149.92 (d, C-6'), 140.49 (d, C-4'), 137.44 (d, C-6), 133.20 (s, C-3'), 128.27 (d, C-5'), 97.61 (d, C-5), 38.38 (q, NCH₃), 34.52 (q, NCH₃); *Anal*. Calcd. for C₁₂H₁₃N₄O₄S (*M*ᵣ=322.34): C (44.71), H (3.75), N (21.73), found: C (44.72), H (3.87), N (21.79).

### EXAMPLE 3. 1-Methanesulfonylcytosine and 4-N-methanesulfonylcytosine

The mixture of cytosine (500 mg, 4.50 mmol) and BSA (3.3 ml, 13.50 mmol) in dry acetonitrile (15 ml) was heated under reflux for 30 minutes. The colorless solution was cooled to 0 °C and MsCI (0.42 ml, 5.40 mmol) was added. The solution was heated under reflux overnight. The solvent was then evaporated and the residue was purified by flash chromatography (methylene chloride:methanol 9:1). 1-Methanesulfonylcytosine (91 mg, 11.5%) and 4-N-methanesulfonylcytosine (226 mg, 26.5%) were isolated as white crystals. The reaction conversion was 38%.

*1-Methanesulfonylcytosine*: mp 222 °C; UV(MeOH): λₘₐₓ/nm 218.1 and 283.7 (log ε 4.29 and 4.45); IR (KBr) νₘₐₓ/cm⁻¹: 3410 (s), 3380 (s), 3100 (s), 2950 (m), 1680-1640 (br, s), 1520 (s), 1485 (s), 1350 (s), 1320 (s), 1290 (s), 1170 (s); ¹H-NMR (d₆-DMSO) δ/ppm: 7.92 (d, 2H, NH₂, *J*=8.7 Hz), 7.86 (d, 1H, H-6, *J*=7.8 Hz), 5.91 (d, 1H, H-5, *J*=7.8 Hz), 3.64 (s, 3H, CH₃); ¹³C-NMR (d₆-DMSO) δ/ppm: 166.23 (s, C-4), 152.20 (s, C-2), 139.26 (d, C-6), 96.76 (d, C-5), 41.16 (q, CH₃); *Anal.* Calcd. for C₅H₇N₃O₃S (*M*ᵣ=189.20): C (31.74), H (3.73), N (22.21), found: C (31.92), H (4.01), N (22.02).

*4-N-Methanesulfonylcytosine*: mp 281-282 °C; UV(MeOH): λₘₐₓ/nm 220.8 and 286.4 (log ε 3.38 and 3.74); IR (KBr) νₘₐₓ/cm⁻¹: 3200 (br, m), 3100 (m), 2930 (m), 1730 (s), 1640 (s), 1565 (s), 1450 (m), 1400 (m), 1380 (s), 1300 (s), 1130 (s); ¹H-NMR (d₆-DMSO) δ/ppm: 11.64 (s, 2H, NH-SO₂- and NH), 7.60 (d, 1H, H-6, *J*=7.6 Hz), 6.35 (d, 1H, H-5, *J*=7.6 Hz), 2.95 (s, 3H, CH₃); ¹³C-NMR (d₆-DMSO) δ/ppm: 160.37 (s, C-4), 149.70 (s, C-2), 144.13 (d, C-6), 95.48 (d, C-5), 42.21 (q, CH₃); *Anal*. Calcd. for C₅H₇N₃O₃S (*M*ᵣ=189.20): C (31.74), H (3.73), N (22.21), found: C (31.92), H (3.71), N (22.12).

### EXAMPLE 4. 5-Bromo-1-p-toluenesulfonylcytosine

A) The mixture of 5-bromocytosine (110 mg, 0.58 mmol) and BSA (0.43 ml, 1.74 mmol) in dry acetonitrile (2 ml) was heated under reflux for 45 minutes. The colorless solution was cooled to 0 °C and TsCl (133 mg, 0.70 mmol) was added. After 30 minutes refluxing, the solvent was evaporated under reduced pressure without external heating. Ethanol was added in the residue and the solid of 5-bromcytosine was filtered. The filtrate was evaporated, and the residue was purified by preparative chromatography (methylene chloride:methanol 9:1), yielding the product (48 mg, 24%) and 1-*p*-toluenesulfonylcytosine (8 mg, 5%).
B) TsCl (300 mg, 1.58 mmol) was added to the suspension of 5-bromocytosine (150 mg, 0.79 mmol) in dry pyridine (8 ml). After stirring at room temperature overnight, the solvent was evaporated under reduced pressure without external heating. Ethanol was added and the solid of 5-bromocytosine filtered. The filtrate was evaporated and the residue purified by preparative chromatography (methylene chloride:methanol 9:1). The product was obtained after recrystallization from methanol (50 mg, 18%), mp 213 °C; UV (MeOH): λₘₐₓ/nm 234.7 and 254.0 (log ε 3.98 and 3.95); IR (KBr) νₘₐₓ/cm⁻¹: 3450 (s), 3070 (m), 2960 (w), 2920 (w), 1680 (s), 1640 (s), 1600 (m), 1490-1475 (s), 1370 (s), 1170 (s); ¹H-NMR (d₆-DMSO) δ/ppm: 8.61 (s, 1H, NH), 8.35 (s, 1H, H-6), 7.95 (d, 2H, Ph, *J*=8.1 Hz), 7.72 (s, 1H, NH), 7.46 (d, 2H, Ph, *J*=8.1 Hz), 2.42 (s, 3H, CH₃); ¹³C-NMR (d₆-DMSO) δ/ppm: 162.85 (s, C-4), 149.94 (s, C-2), 146.08 (s, Ph), 139.74 (d, C-6), 133.83 (s, Ph), 129.89 (d, Ph), 129.44 (d, Ph), 90.27 (s, C-5), 21.27 (q, CH₃); *Anal*. Calcd. for C₁₁H₁₀N₃O₃SBr (*M*ᵣ=344.19): C (38.39), H (2.93), N (12.21), found: C (38.19), H (2.94), N (12.44).

### EXAMPLE 5. 5-Iodo-1-p-toluenesulfonylcytosine

A) The mixture of 5-iodocytosine (250 mg, 1.06 mmol) and BSA (0.78 ml, 3.17 mmol) was heated under reflux in dry acetonitrile (3.5 ml) for 45 minutes. In the obtained red solution, TsCI (242 mg, 1.27 mmol) was added. After heating under reflux for 45 minutes, the solvent was evaporated and the residue was purified by preparative chromatography (methylene chloride:methanol 9:1). The product was obtained after recrystallization from methanol (20 mg, 5%), mp 210 °C; and 1-*p*-toluenesulfonylcytosine, 8 mg (3%) was also isolated.
B) TsCl (322 mg, 1.69 mmol) was added to the suspension of 5-iodocytosine (200 mg, 0.84 mmol) in dry pyridine (9 ml). After stirring at room temperature overnight, the solvent was evaporated under reduced pressure without external heating. Ethanol was added to the remaining oil and the white solid of the product was filtered and recrystallized from ethanol (171 mg, 52%), UV (MeOH): λₘₐₓ/nm 228.9 and 289.4 (log ε 4.15 and 3.67); IR (KBr) νₘₐₓ/cm⁻¹: 3480 (m), 3310 (m), 3200 (w), 2920 (vw), 2850 (vw), 1640 (s), 1560 (s), 1525 (m), 1390 (s), 1370 (s), 1190 (s), 1170 (s); ¹H-NMR (d₆-DMSO) δ/ppm: 9.18 (s, 1H, NH), 8.82 (s, 1H, NH), 8.25 (s, 1H, H-6), 7.53 (d, 2H, Ph, *J*=8.0 Hz), 7.16 (d, 2H, Ph, *J*=7.8 Hz), 2.31 (s, 3H, CH₃); ¹³C-NMR (d₆-DMSO) δ/ppm: 160.32 (s, C-4), 153.52 (d, C-6), 142.55 (s, C-2), 147.51 (s, Ph), 138.57 (s, Ph), 128.56 (d, Ph), 125.76 (d, Ph), 54.99 (s, C-5), 20.91 (q, CH₃). *Anal*. Calcd. for C₁₁H₁₀N₃O₃SI (*M*ᵣ=391.19): C (33.77), H (2.58), N (10.74), found. C (33.94), H (2.75), N (10.71).

### EXAMPLE 6. 4-N-Methanesulfonyl-1-p-toluenesulfonylcytosine

A) MsCl (0.029 ml, 0.38 mmol) was added to the cold suspension of 1-*p*-toluenesulfonylcytosine (50 mg, 0.19 mmol) in dry pyridine (2 ml). After stirring at room temperature overnight, the solvent was evaporated under reduced pressure. Ethanol was added to the remaining oil and the white solid of theproduct was filtered and recrystallized from ethanol (20 mg, 31%).
B) TsCl (127 mg, 0.67 mmol) was added to the suspension of 4-*N*-methanesulfonylcytosine (63 mg, 0.33 mmol) in dry pyridine (3.5 ml). After stirring at room temperature overnight, the solvent was evaporated under reduced pressure. Ethanol was added to the remaining oil and the white solid of the product was filtered and recrystallized from ethanol (11 mg). The residue was purified by preparative chromatography (methylene chloride:methanol 20:1), and additional 30 mg of the product were isolated. Overall yield was 38.5%; mp=229 °C; UV (MeOH): λₘₐₓ/nm 234.4 and 276.5 (log ε 3.51 and 3.68); IR (KBr) νₘₐₓ/cm⁻¹: 3200 (m), 3100 (m), 2920 (m), 2850 (m), 1730 (s), 1640-1630 (s), 1570 (m), 1460 (m), 1410-1390 (m), 1280 (s), 1175 (s), 1115 (m); ¹H-NMR (d₆-DMSO) δ/ppm: 12.28 (brs, 1H, NH), 8.26 (d, 1H, H-6, *J*=8.4 Hz), 7.93 (d, 2H, Ph, *J*=8.1 Hz), 7.51 (d, 2H, Ph, *J*=8.1 Hz), 6.69 (d, 1H, H-5, *J*=8.4 Hz), 3.02 (s, 3H, CH₃), 2.44 (s, 3H, CH₃); ¹³C-NMR (d₆-DMSO) δ/ppm: 158.86 (s, C-4), 146.78 (s, C-2), 146.22 (s, Ph), 139.06 (d, C-6), 132.68 (s, Ph), 130.03 (d, Ph), 129.29 (d, Ph), 98.65 (s, C-5), 41.00 (q, CH₃), 21.33 (q, CH₃). *Anal*. Calcd. or C₁₂H₁₃N₃O₅S₂ (*M*ᵣ=343.39): C (41.97), H (3.82), N (12.24), found: C (41.98), H (4.00), N (12.06).

### EXAMPLE 7. 1-Methansulfonyl-4-N-p-toluenesulfonylcytosine

A) The mixture of 1-methanesulfonylcytosine (32 mg, 0.17 mmol) and BSA (0.084 ml, 0.34 mmol) was heated under reflux in dry acetonitrile (1 ml) for 45 minutes. TsCl (39 mg, 0.20 mmol) was added, and after heating under reflux for 2 hours, the solvent was evaporated and the residue was purified by preparative chromatography (methylene chloride:methanol 3:1). After recrystallization from ethanol, the white crystals of the product were obtained (28 mg, 48%).
B) TsCl (63 mg, 0.33 mmol) was added to the suspension of 1-methanesulfonylcytosine (52 mg, 0.28 mmol) in dry pyridine (3 ml). After stirring at room temperature overnight, the solvent was evaporated under reduced pressure. Ethanol was added to the remaining oil and the white solid of the product was filtered and recrystallized from ethanol (85 mg, 90%); mp=206-208 °C; UV (MeOH): λₘₐₓ/nm 234.5 and 277.2 (log ε 3.89 and 4.06); IR (KBr) νₘₐₓ/cm⁻¹: 3220 (m), 3100 (w), 2920 (w), 2850 (w), 1730 (s), 1640 (s), 1590-1570 (s, doublet), 1480-1450 (s, br), 1400 (s, doublet), 1370 (s), 1330 (s), 1275-1255 (s, doublet), 1190 (s), 1170 (s); ¹H-NMR (d₆-DMSO) δ/ppm: 12.30 (s, 1H, NH), 8.27 (d, 1H, H-6, *J*=8.4 Hz), 7.94 (d, 2H, Ph, *J*=8.1 Hz), 7.51 (d, 2H, Ph, *J*=8.1 Hz), 6.69 (d, 1H, H-5, *J*=8.4 Hz), 3.03 (s, 3H, CH₃), 2.44 (s, 3H, CH₃); ¹³C-NMR (d₆-DMSO) δ/ppm: 158.99 (s, C-4), 146.98 (s, C-2), 146.36 (s, Ph), 139.26 (d, C-6), 132.84 (s, Ph), 130.20 (d, Ph), 129.45 (d, Ph), 98.70 (s, C-5), 42.39 (q, CH₃), 21.30 (q, CH₃). *Anal*. Calcd. for C₁₂H₁₃N₃O₅S₂ (*M*ᵣ=343.39): C (41.97), H (3.82), N (12.24), found: C (42.01), H (3.47), N (12.46).

### EXAMPLE 8. 1,4-N-Dimethanesulfonylcytosine

MsCl (0.7 ml, 9.00 mmol) was added to the cold suspension of cytosine (500 mg, 4.50 mmol) in dry pyridine (15 ml). After stirring at room temperature overnight, the solvent was evaporated under reduced pressure. Ethanol was added to the remaining oil and the white solid of the product was filtered and recrystallized from ethanol (825 mg, 69%); mp=220 °C; UV (MeOH): λₘₐₓ/nm 220.0 and 273.2 (log ε 3.84 and 4.19); IR (KBr) νₘₐₓ/cm⁻¹: 3260 (m), 3030 (w), 2940 (w), 1790-1760 (s), 1640 (s), 1575 (m), 1465-1455 (m, doublet), 1390 (s), 1350 (s), 1290-1260 (s), 1150 (s), 1125 (s); ¹H-NMR (d₆-DMSO) δ/ppm: 12.40 (s, 1H, NH), 7.99 (d, 1H, H-6, *J*=8.4 Hz), 6.64 (d, 1H, H-5, *J*=8.4 Hz), 3.72 (s, 3H, CH₃), 3.04 (s, 3H, CH₃); ¹³C-NMR (d₆-DMSO) δ/ppm: 163.98 (s, C-4), 152.38 (s, C-2), 144.20 (d, C-6), 102.98 (s, C-5), 47.27 (q, CH₃), 46.39 (q, CH₃). *Anal.* Calcd. for C₆H₉N₃O₅S₂ (*M*ᵣ=267.29): C (26.96), H (3.39), N (15.72), found: C (27.05), H (3.30), N (15.61).

### EXAMPLE 9. 1,4-N-Di-p-toluenesulfonylcytosine

A) TsCl (185 mg, 0.97 mmol) was added to the suspension of 1-*p*-toluenesulfonylcytosine (200 mg, 0.81 mmol) in dry pyridine (8.5 ml). After stirring at room temperature overnight, the solvent was evaporated under reduced pressure. The remaining oil was purified by preparative chromatography (methylene chloride/methanol 9:1). After recrystallisation from ethanol the product was obtained (295 mg, 87%); mp=182 °C; UV (MeOH): λₘₐₓ/nm 227.2 and 283.0 (log ε 4.39 and 4.45); IR (KBr) νₘₐₓ/cm⁻¹: 3200 (w), 3090 (w), 3030 (w), 2920 (w), 2850 (w), 1730-1715 (s, doublet), 1630 (s), 1570 (s, doublet), 1450 (m, doublet), 1400 (s, doublet), 1370 (m), 1290 (s), 1260 (m), 1175 (s), 1155 (s); ¹H-NMR (d₆-DMSO) δ/ppm: 12.10 (brs, 1H, NH), 8.26 (d, 1H, H-6, *J*=8.4 Hz), 6.76 (d, 1H, H-5, *J*=8.4 Hz), 7.93 (d, 2H, Ph, *J*=8.1 Hz), 7.73 (d, 2H, Ph, *J*=8.1 Hz), 7.49 (d, 2H, Ph, *J*=8.1 Hz), 7.37 (d, 2H, Ph, *J*=8.1 Hz), 2.43 (s, 3H, CH₃), 2.38 (s, 3H, CH₃); ¹³C-NMR (d₆-DMSO) δ/ppm: 159.79 (s, C-4), 146.79 (s, Ph), 146.42 (s, C-2), 142.94 (s, Ph), 139.52 (d, C-6), 132.91 (s, Ph), 132.90 (s, Ph), 130.06 (d, Ph), 129.63 (d, Ph), 129.38 (d, Ph), 126.47 (d, Ph), 98.78 (s, C-5), 21.21 (q, CH₃), 20.95 (q, CH₃). *Anal*. Calcd. for C₁₈H₁₇N₃O₅S₂ (*M*ᵣ=419.48): C (51.54), H (4.09), N (10.02), found: C (51.26), H (4.20), N (10.00).
B) TsCl (343 mg, 1.80 mmol) was added to the suspension of cytosine (100 mg, 0.90 mmol) in dry pyridine (9.5 ml). After stirring at room temperature for 48 hours, the solvent was evaporated under reduced pressure. The remaining oil was purified by preparative chromatography (methylene chloride:methanol 15:1). After recrystallisation from ethanol, 106 mg (44.4%) of 1-p-toluenesulfonylcytosine and 92 mg (24.4%) 1,4-*N*-di-*p*-toluenesulfonylcytosine were obtained.

### EXAMPLE 10. Growth inhibitory effects

Compounds 1 and 2 as shown in Scheme (2) were tested on anticancer activity *in vitro* by using human tumor pancreatic carcinoma (MiaPaCa 2), human cervix carcinoma (HeLa), laryngeal carcinoma (Hep 2) and normal human fibroblasts (Hef 2) cell lines.

*Cell lines.* Human tumor pancreatic carcinoma cells (MiaPaCa 2), laryngeal carcinoma (Hep 2), human cervical carcinoma (HeLa) and normal human fibroblasts (Hef 2) were grown as monolayers at 37 °C in a humidified atmosphere with 5% CO₂ in DMEM supplemented with 10% fetal calf serum, 2 mM glutamine, 100 U/ml penicillin and 100 mg/ml streptomycin. They were routinely checked for mycoplasma contamination by Hoechst 33250 fluorochrome staining and by aerobic and anaerobic culture techniques.

*Cell Cytotoxicity.* To the purpos of the experiments the cells (200 ml; 2x10⁴ cells/ml) were plated onto 96-microwell plates. After 24 hours, test compounds at different concentrations (10⁻⁴-10⁻⁸ M), were added to the cells. Three days later, cell viability was determined by trypan blue exclusion assay. The control cells were grown under same conditions without addition of test compounds. The cell growth as determined by MTT staining procedures (Mickisch *et al*., 1989). This method depends on the conversion of a water-soluble tetrazolium salt (MTT) to a purple colored formazan precipitate. The reaction affected by dehydrogenase enzymes active only in living cells. Tests were carried out in 96-well microculture plates. 10⁴ viable tumor cells per well at 4 h incubation time 20 µg MTT7100 µl total medium volume. Formazan crystals were dissolved in DMSO and the plates were immediately measured on a microculture plate reader at 540 nm.

*Results.* The growth inhibitory effects of investigated substances 1 and 2 Scheme (2), on different human cancer and normal cell lines are shown in Tables I, II, III and IV.

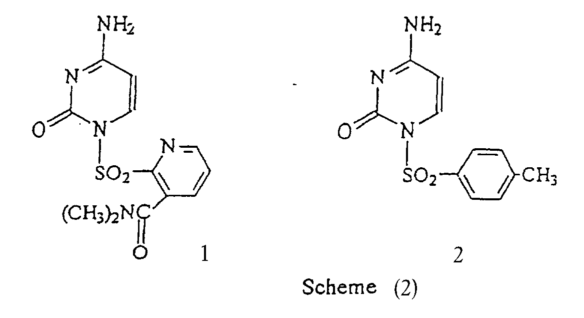

On HeLa cells (human cervical carcinoma) very strong inhibitory effect was found for substances 1 and 2 at concentration of 10⁻⁵ M. This concentration inhibited the cell growth for 85% (Table I).

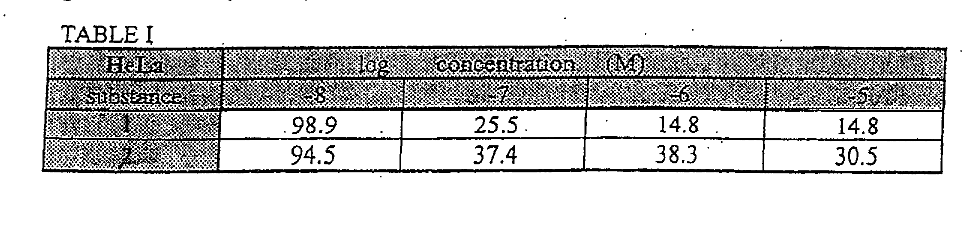

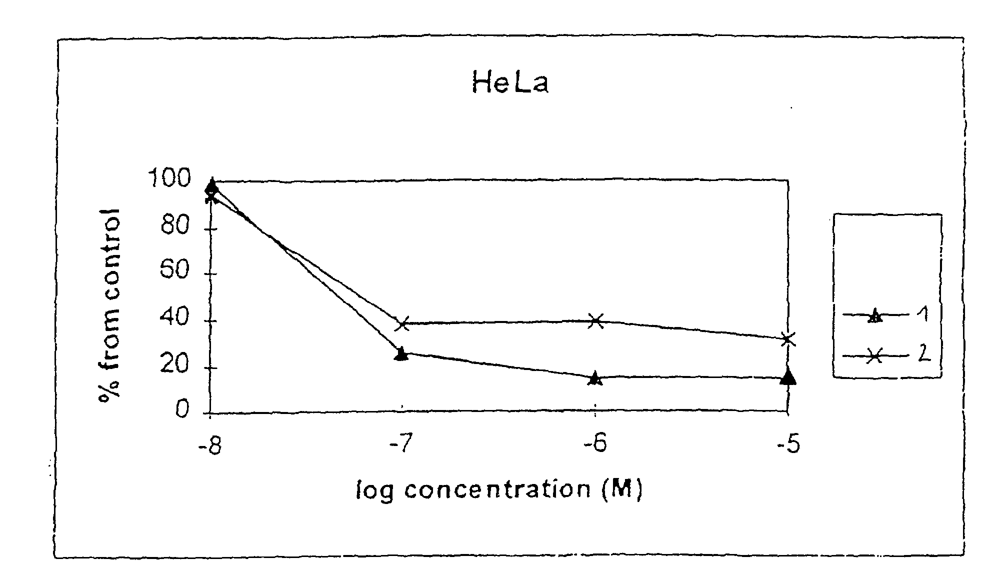

The most pronounced growth inhibitory effect on Hep 2 (laryngeal carcinoma) was observed for substance 1, applying concentration 10⁻⁵ M. This concentration inhibited growth for 80%. The substance 2 showed less pronounced inhibitory effects of only 20%, at concentration of 10⁻⁵ M (Table II).

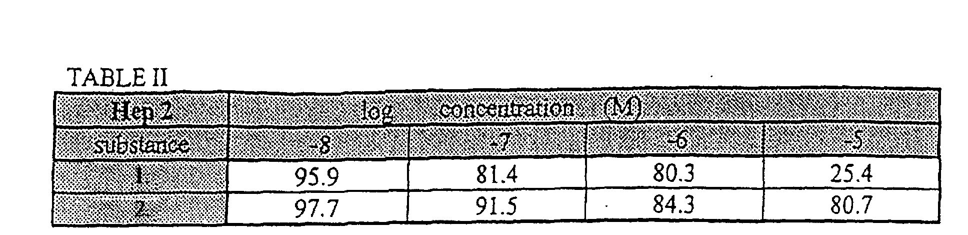

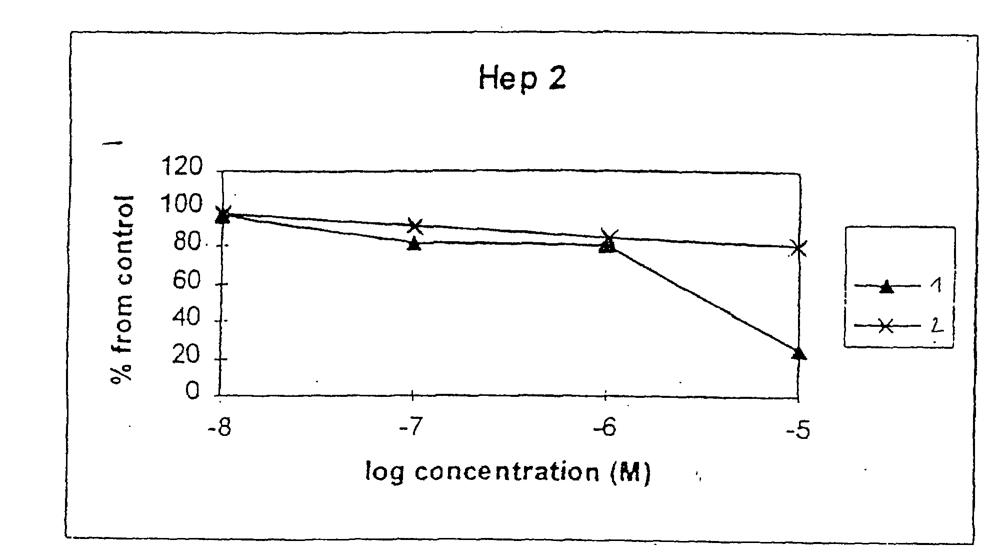

On MiaPaCa 2 (pancreatic carcinoma) the inhibitory effect on cell growth was observed at concentration of 10⁻⁵ M. The substance **1** inhibits the cell growth for 40%. and substance 2 for 65% (Table III).

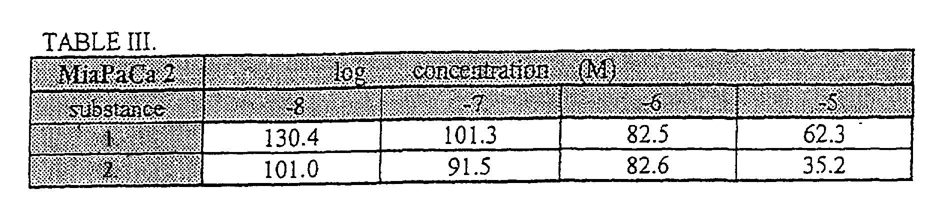

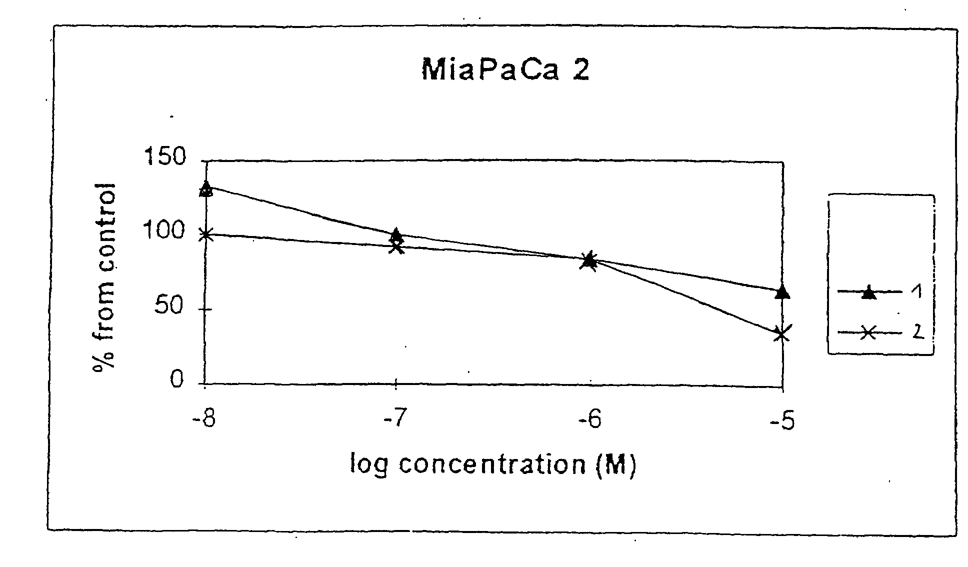

The inhibitory effects of the investigated sulfonyl derivatives on normal human cell lines are weak in comparison to cancer cells. The concentration 10⁻⁵ M of all investigated substances inhibits the cell growth for more than 50% of tumor cell lines (MiaPaCa 2, HeLa), but effect on normal cell lines was less than 40% (Table IV).

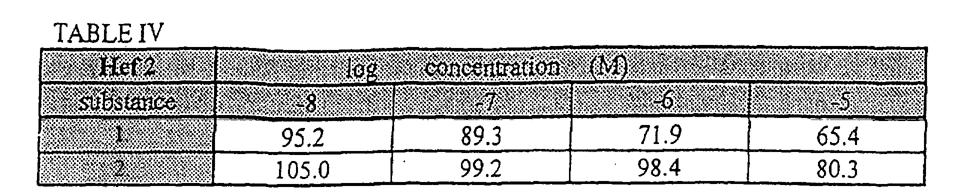

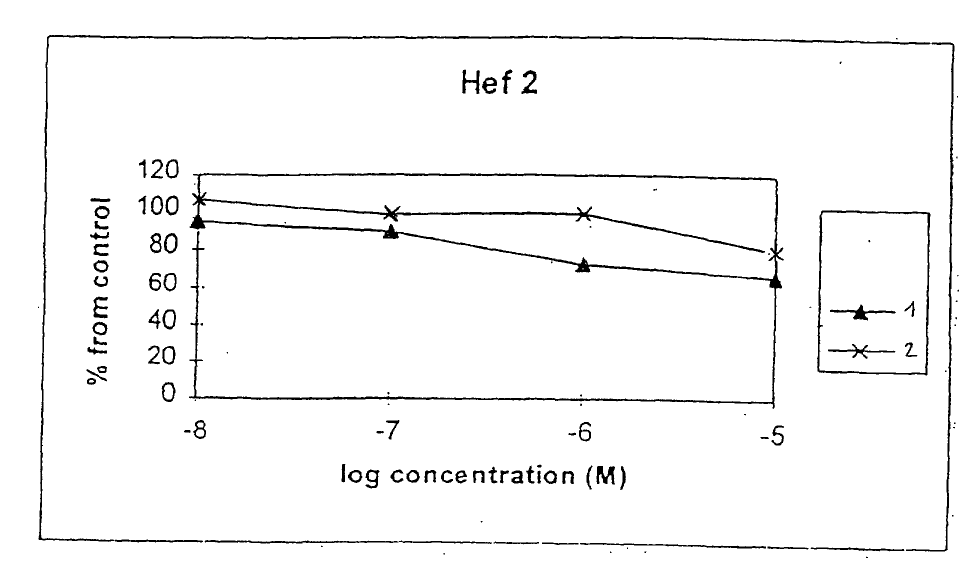

The most efficient antiproliferative effects of investigated sulfonyl derivatives ( 1 and 2) were achieved at concentration of 10⁻⁵ M. The cell growth inhibitory effects of the investigated sulfonylurea derivatives were found much weaker on normal human fibroblasts compared to cancer cells. For example, substance **1** produced 75% growth inhibition of Hep 2, and only 35% growth inhibition of Hef 2. Also compound 2 inhibited the growth for 70% of HeLa and MiaPaCa 2 cells and only 20% of Hef 2.

### 6) APPLYING THE INVENTION

This invention relates to processes for the preparation of yet unknown N-1 sulfonyl and N-1,NH-4 disulfonyl derivatives of pyrimidine nucleo bases (cytosine) and their modifications (general formulae II, . Substances 1 and 2 shown on Scheme (5) show *in vitro* anticancer activity. Compounds shown in this invention are of interest for anticancer drugs development.

Hence, it is another object of the present invention to use a sulfonyl pyrimidine derivative of general formula II as described hereinbefore for preparing a pharmaceutical composition for treating cancer.

## Claims

1. Compounds of general formula II 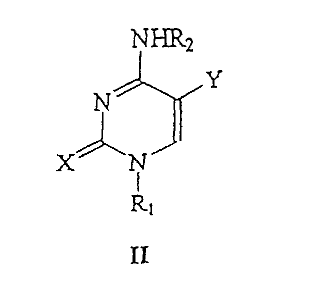 wherein
X = O, S;
Y = H, F, Cl, Br, I, C₁-C₅ alkyl, NO₂, NH₂, substituted alkynyl
(C≡C**R**_{**4**}**; R**_{**4**} = H, Si(CH₃)₃, C₁-C₃ alkyl, halogen, CO-alkyl); substituted alkenyl (CH=CH**R**₅; R₅ = H, F, Cl, Br, I, C₁-C₃ alkyl , CN, COCH₃, CO₂CH₃) ;
**R**_{**1**} = SO_{**2**}**R**_{**6**;}
**R**_{**2**} = H or SO_{**2**}**R**_{**6**}, in which **R**_{**6**} is the same or different when
**R**_{**1**} = SO_{**2**}**R**_{**6**} and **R**_{**2**} = SO_{**2**}**R**_{**6**}
**R**_{**6**} = C₁-C₃ (branched) alkyl, C₁-C₃ haloalkyl, C₁-C₃ alkyl substituted with C₁-C₃ (halo)alkoxy group, C₂-acyl,

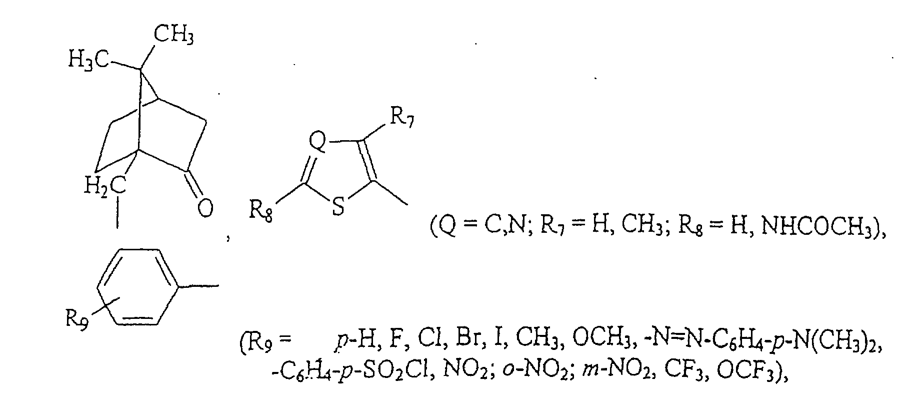

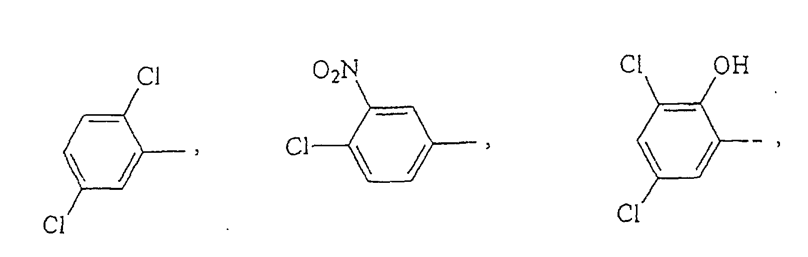

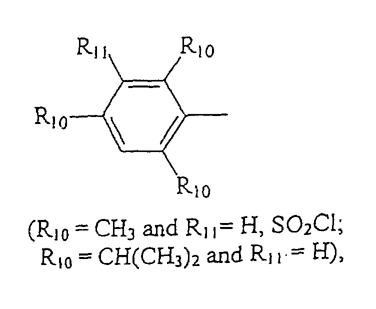

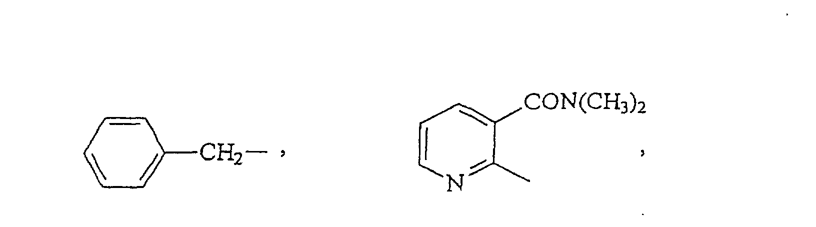

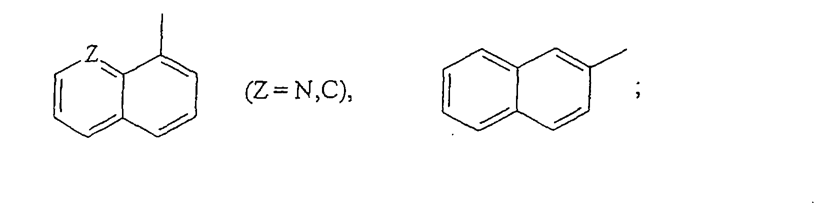

2. The compound of claim 1, 1-*p*-toluenesulfonylcytosine (4-amino-1-*p*-toluenesulfonylpyrimidine-2-one).

3. The compound of claim 1, 1,4-*N*-di-*p*-toluenesulfonylcytosine (4-*N*-*p*-toluenesulfonylamino-1-*p*-toluenesulfonylpyrimidine-2-one).

4. The compound of claim 1, 1-(2-sulfonyl-3-*N,N*-dimethylaminocarbonylpyridine)cytosine (1-(2-sulfonyl-3-*N*,*N*-dimethylaminocarbonylpyridine)pyrimidine-2-one).

5. The compound of claim 1, 1-methanesulfonylcytosine (4-amino-1-methanesulfonylpyrimidine-2-one).

6. The compound of claim 1, 4-*N*-methanesulfonylcytosine (4-*N*-methanesulfonylaminopyrimidine-2(1H)-one).

7. The compound of claim 1, 1,4-*N*-dimethanesulfonylcytosine (4-*N*-methanesulfonyl-1-methanesulfonylpyrimidine-2-one).

8. The compound of claim 1, 4-*N*-methanesulfonyl-1-*p*-toluenesulfonylcytosine (4-*N*-methanesulfonylamino-1-*p*-toluenesulfonylpyrimidine-2-one).

9. The compound of claim 1, 1-methanesulfonyl-4-*N*-*p*-toluenesulfonylcytosine (1-methanesulfonyl-4-*N*-*p*-toluenesulfonylaminopyrimidine-2-one).

10. The compound of claim 1, 5-bromo-1-*p*-toluenesulfonyl-cytosine (4-amino-5-bromo-1-*p*-toluenesulfonylpyrimidine-2-one).

11. The compound of claim 1, 5-iodo-1-*p*-toluenesulfonylcytosine (4-amino-5-iodo-1-*p*-toluenesulfonylpyrimidine-2-one).

12. The process for the preparation of sulfonyl pyrimidine derivatives of general formula II as defined in claim 1 wherein sulfonyl chlorides of the formula R₆-SO₂Cl, wherein R₆ has the meaning as given in claim 1, are reacted with properly activated pyrimidine nucleo base derivatives II in ratio of 1.2-2.0 equivalents of R₆-SO₂Cl in an organic solvent for 0.5-24 hours at temperature of 20-80°C.

13. The process for the preparation of sulfonyl pyrimidine derivatives of general formula II as defined in claim 1 wherein sulfonyl chlorides of the formula R₆-SO₂Cl, wherein R₆ has the meaning as given in claim 1, are reacted with properly activated pyrimidine nucleo base derivatives II in ratio of 1.2-2.0 equivalents of R₆-SO₂Cl in pyridine as organic solvent for 1-48 hours at temperature of 20°C.

14. The use of a sulfonyl pyrimidine derivative of general formula II according to any of claims 1-11 for preparing a pharmaceutical composition for treating cancer.

## Patentansprüche

1. Verbindungen der allgemeinen Formel II 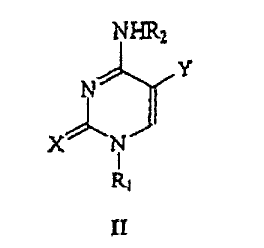 wobei
X = O, S;
Y = H, F, Cl, Br, I, C₁-C₅ Alkyl, NO₂, NH₂, substituiertes Alkynyl (C=CR₄; R₄ = H, Si(CH₃)₃, C₁-C₃ Alkyl, Halogen, CO-Alkyl), substituiertes Alkenyl (CH=CHR₅; R₅ = H, F, Cl, Br, I, C₁-C₃ Alkyl, CN, COCH₃, CO₂CH₃) ;
R₁ = SO₂R₆;
R₂ = H oder SO₂R₆, worin R₆ das gleiche oder verschieden ist, wenn R₁ = SO₂R₆ und R₂ = SO₂R₆;
R₆ = C₁-C₃ (verzweigtes) Alkyl, C₁-C₃ Haloalkyl, mit einer C₁-C₃ (Halo)Alkoxy-Gruppe substituiertes C₁-C₃ Alkyl, C₂-Acyl,

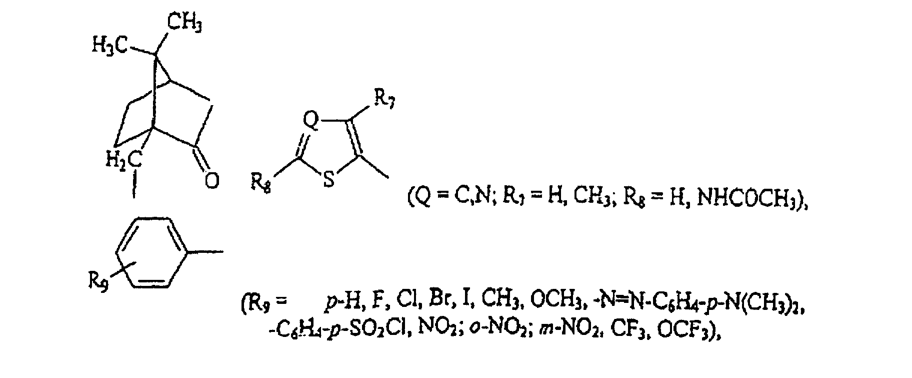

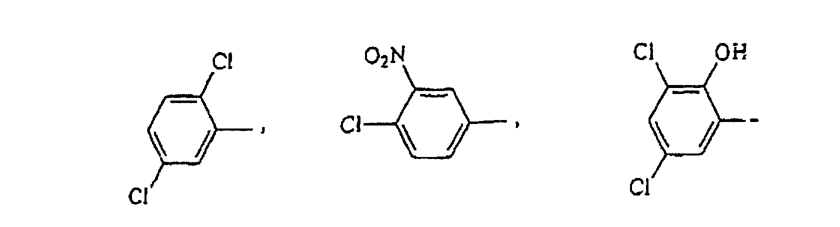

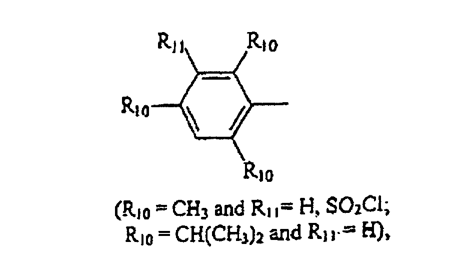

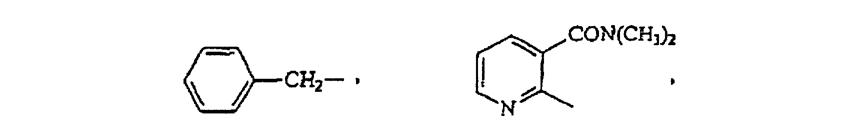

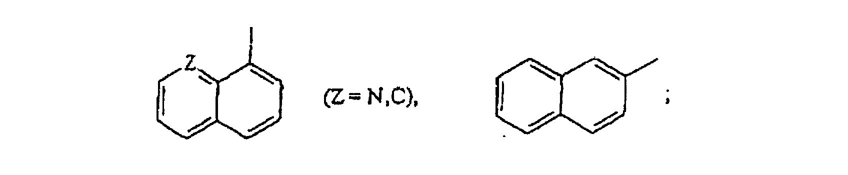

2. Verbindung gemäß Anspruch 1, 1-*p*-Toluolsulfonylcytosin (4-Amino-1-*p*-toluolsulfonylpyrimidin-2-on).

3. Verbindung gemäß Anspruch 1, 1,4-*N*-Di-*p*toluolsulfonylcytosin (4-*N*-*p*-Toluolsulfonylamino-1-*p*toluolsulfonylpyrimidin-2-on).

4. Verbindung gemäß Anspruch 1, 1-(2-Sulfonyl-3-*N,N*-dimethylaminocarbonylpyridin)cytosin (1-(2-Sulfonyl-3-*N,N*-dimethylaminocarbonylpyridin)pyrimidin-2-on).

5. Verbindung gemäß Anspruch 1, 1-Methansulfonylcytosin (4-Amino-1-methansulfonylpyrimidin-2-on).

6. Verbindung gemäß Anspruch 1, 4-*N*-Methansulfonylcytosin (4-*N*-Methansulfonylaminopyrimidin-2(1H)-on).

7. Verbindung gemäß Anspruch 1, 1,4-*N*-Dimethansulfonylcytosin (4-*N*-Methansulfonyl-lmethansulfonylpyrimidin-2-on).

8. Verbindung gemäß Anspruch 1, 4-*N*-Methansulfonyl-1-*p*toluolsulfonylcytosin (4-*N*-Methansulfonylamino-1-*p*toluolsulfonylpyrimidin-2-on).

9. Verbindung gemäß Anspruch 1, 1-Methansulfonyl-4-*N*-*p*toluolsulfonylcytosin (1-Methansulfonyl-4-*N*-*p*toluolsulfonylaminopyrimidin-2-on).

10. Verbindung gemäß Anspruch 1, 5-Brom-1-*p*toluolsulfonyl-cytosin (4-Amino-5-brom-1-*p*toluolsulfonylpyrimidin-2-on).

11. Verbindung gemäß Anspruch 1, 5-Iod-1-*p*toluolsulfonyl-cytosin (4-Amino-5-iod-1-*p*toluolsulfonylpyrimidin-2-on).

12. Verfahren zur Herstellung von Sulfonylpyrimidin-Derivaten der allgemeinen Formel II wie in Anspruch 1 definiert, wobei Sulfonylchloride der Formel R₆-SO₂Cl, wobei R₆ die Bedeutung besitzt wie in Anspruch 1 angegeben, mit geeignet aktivierten Pyrimidin-Nukleobase-Derivaten II im Verhältnis von 1,2-2,0 Äquivalenten R₆-SO₂Cl in einem organischen Lösungsmittel für 0,5-24 Stunden bei einer Temperatur von 20-80°C umgesetzt werden.

13. Verfahren zur Herstellung von Sulfonylpyrimidin-Derivaten der allgemeinen Formel II wie in Anspruch 1 definiert, wobei Sulfonylchloride der Formel R₆-SO₂Cl, wobei R₆ die Bedeutung besitzt wie in Anspruch 1 angegeben, mit geeignet aktivierten Pyrimidin-Nukleobase-Derivaten II im Verhältnis von 1,2-2,0 Äquivalenten R₆-SO₂Cl in Pyrimidin als organischem Lösungsmittel für 1-48 Stunden bei einer Temperatur von 20°C umgesetzt werden.

14. Verwendung eines Sulfonylpyrimidin-Derivats der allgemeinen Formel II gemäß einem der Ansprüche 1-11 zur Herstellung einer pharmazeutischen Zusammensetzung zur Behandlung von Krebs.

## Revendications

1. Composés de formule générale II 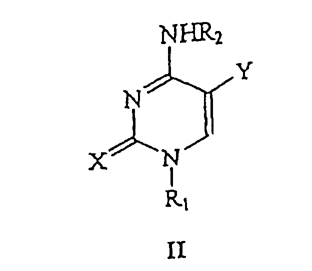 dans laquelle :
X = O, S ;
Y = H, F, Cl, Br, I, alkyle en C₁-C₅, NO₂, NH₂, alkynyle substitué ;
(C≡CR₄ ; R₄ = H, Si(CH₃)₃,alkyle en C₁-C₃, halogène, CO-alkyle) ;
alcényle substitué (CH=CHR₅ ; R₅ = H, F, Cl, Br, I, alkyle en C₁-C₃, CN, COCH₃, CO₂CH₃) ;
R₁ = SO₂R₆;
R₂ = H ou SO₂R₆, où R₆ est identique ou différent lorsque R₁ = SO₂R₆ et R₂ = SO₂R₆ ;
R₆ = alkyle en C₁-C₃ (ramifié), haloalkyle en C₁-C₃, alkyle en C₁-C₃ substitué par un groupe (halo)alcoxy en C₁-C₃, C₂-acyle,

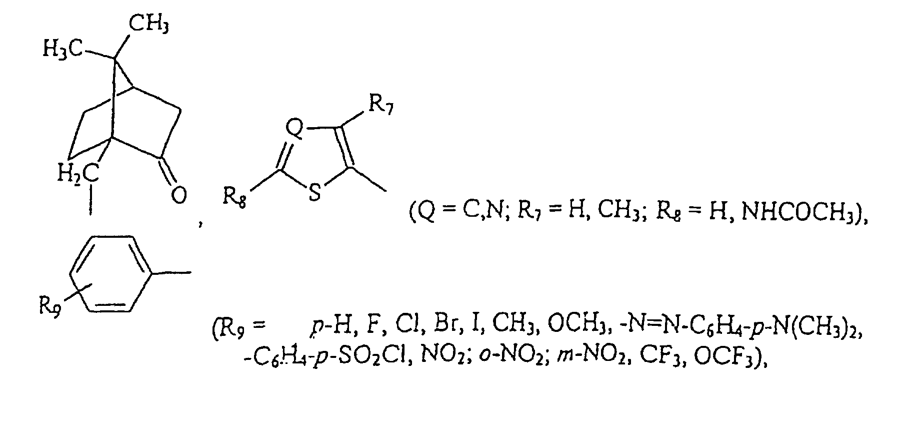

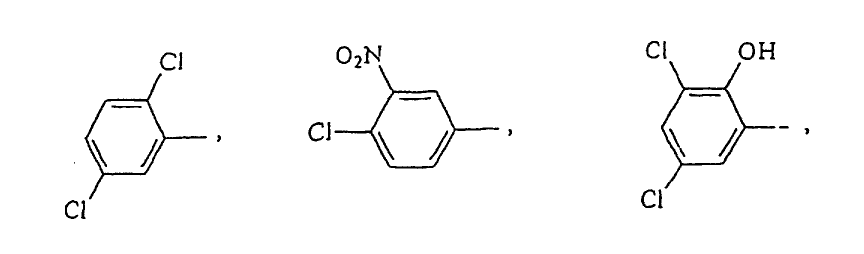

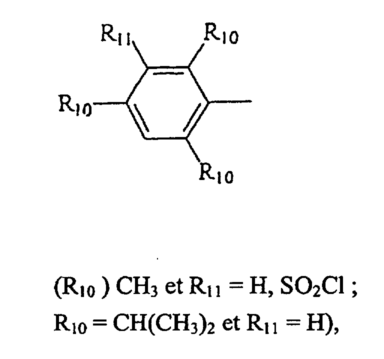

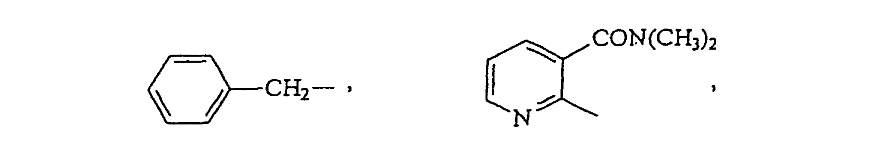

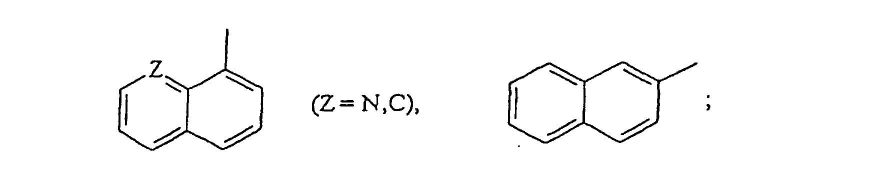

2. Le composé selon la revendication 1, la 1-*p*-toluènesulfonylcytosine (4-amino-1-*p*-toluènesulfonylpyrimidine-2-one).

3. Le composé selon la revendication 1, la 1,4-*N*-di-*p*-toluènesulfonylcytosine (4-*N-p*-toluènesulfonylamino-1-*p*-toluènesulfonylpyrimidine-2-one).

4. Le composé selon la revendication 1, la 1-(2-sulfonyl-3-*N,N*-diméthylaminocarbonylpyridine)cytosine (1-(2-sulfonyl-3-*N*,*N*-diméthylaminocarbonylpyridine) pyrimidine-2-one).

5. Le composé selon la revendication 1, la 1-méthanesulfonylcytosine (4-amino-1-méthanesulfonylpyrimidine-2-one).

6. Le composé selon la revendication 1, la 4-*N*-méthanesulfonylcytosine (4-*N*-méthanesulfonylaminopyrimidine-2(1H)-one).

7. Le composé selon la revendication 1, la 1,4-*N*-diméthanesulfonylcytosine (4-*N*-méthanesulfonyl-1-méthanesulfonylpyrimidine-2-one).

8. Le composé selon la revendication 1, la 4-*N*-méthanesulfonyl-1-*p*-toluènesulfonylcytosine (4-*N*-méthanesulfonylamino-1-*p*-toluènesulfonylpyrimidine-2-one).

9. Le composé selon la revendication 1, la 1-méthanesulfonyl-4-*N*-*p*-toluène-sulfonylcytosine (1-méthanesulfonyl-4*-N-p*-toluènesulfonylaminopyrimidine-2-one).

10. Le composé selon la revendication 1, 1a 5-bromo-1-*p*-toluènesulfonylcytosine (4-amino-5-bromo-1-*p*-toluènesulfonylpyrimidine-2-one).

11. Le composé selon la revendication 1, la 5-iodo-1-*p*-toluènesulfonylcytosine (4-amino-5-iodo-1-*p*-toluènesulfonylpyrimidine-2-one).

12. Le procédé de préparation des dérivés de sulfonyl pyrimidine de formule générale II comme définie dans la revendication 1, dans lequel on fait réagir des chlorures de sulfonyle de formule R₆-SO₂Cl, où R₆ a la signification indiquée dans la revendication 1, avec des dérivés de base nucléique pyrimidine convenablement activés II selon un rapport de 1,2-2,0 équivalents de R₆-SO₂Cl dans un solvant organique pendant 0,5-24 heures à une température de 20-80 °C.

13. Le procédé de préparation des dérivés de sulfonyl pyrimidine de formule générale II comme défini selon la revendication 1, dans lequel on fait réagir des chlorures de sulfonyle de formule R₆-SO₂Cl, où R₆ a la signification donnée dans la revendication 1, avec des dérivés de base nucléique pyrimidine activés convenablement II selon un rapport de 1,2-2,0 équivalents de R₆-SO₂Cl, dans la pyridine en tant que solvant organique pendant 1 à 48 heures à la température de 20 °C.

14. L'utilisation d'un dérivé de sulfonyl pyrimidine de formule générale II selon l'une quelconque des revendications 1 à 11 pour la préparation d'une composition pharmaceutique pour le traitement du cancer.
